Europäisches Patentamt

European Patent Office

Office européen des brevets

(11) Veröffentlichungsnummer: **0 251 250 B1**

(12) **EUROPÄISCHE PATENTSCHRIFT**

(45) Veröffentlichungstag der Patentschrift: 28.08.91

(21) Anmeldenummer: 87109237.5

(22) Anmeldetag: 26.06.87

(51) Int. Cl.⁵: **A61K 7/08**, A61K 7/13, C07C 309/07

(54) **Haarbehandlungsmittel.**

(30) Priorität: 04.07.86 DE 3622439

(43) Veröffentlichungstag der Anmeldung:
07.01.88 Patentblatt 88/01

(45) Bekanntmachung des Hinweises auf die
Patenterteilung:
28.08.91 Patentblatt 91/35

(84) Benannte Vertragsstaaten:
AT BE CH DE ES FR GB IT LI NL SE

(56) Entgegenhaltungen:
EP-A- 0 177 071
EP-A- 0 184 785
FR-A- 2 353 527
US-A- 2 979 465
US-A- 3 827 497

SOAP/COSMETICS/CHEMICAL SPECIALTIES,
Band 63, Nr. 2, Februar 1987, Seiten 41,42,57,
New York, NY, US; R.A. JACOBS: "Novel anionic sulfonates"

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Höffkes, Horst, Dr.
Carlo-Schmid-Strasse 113
W-4000 Düsseldorf-Hellerhof(DE)**
Erfinder: **Lange, Fritz, Dr.
Baderweg 82
W-4300 Essen(DE)**
Erfinder: **Giede, Karl
Schlehenweg 12
W-4010 Hilden(DE)**

## Beschreibung

Gegenstand der Erfindung sind wässrige kosmetische Haarbehandlungsmittel mit anionischem Tensid, bevorzugt mit saurem PH-Wert, die als anionisches Tensid ein Alkylethersulfonat enthalten.

Viele wässrige Haarbehandlungsmittel enthalten Tenside, um eine bessere Benetzung des Haars, eine Emulgierung oder Dispergierung wasserlöslicher Komponenten, eine gleichmäßigere Verteilung der Komponenten auf dem Haar oder gar um eine Schaum-und Waschwirkung zu erreichen. Von den Tensiden kommt den anionischen Tensiden wegen des guten Schaum- und Waschvermögens, der guten Netz- und Dispergierwirkung die größte Bedeutung zu. Besonders die Alkylethersulfate haben wegen dieser guten Anwendungseigenschaften und da sie sich auch in verdünnter wässriger Lösung durch Zusatz von Elektrolyten stark verdicken lassen, große Bedeutung erlangt. Sie haben jedoch den Nachteil, daß sie bei längerer Lagerung, insbesondere bei pH-Werten unterhalb 5 hydrolytisch gespalten werden, eine Reaktion, die sich infolge der Bildung von Schwefelsäure autokatalytisch beschleunigt, wenn sie einmal begonnen hat. Bekannte hydrolysestabile Tenside, z.B. nichtionogene Ethyloxidaddukte, sind nicht ausreichend schaumbildend, andere, z.B. die anionischen Alkylbenzolsulfonate, sind für kosmetische Anwendungen nicht genügend hautverträglich, wieder andere, z.B. die Alkansulfonate und die Alpha-Olefinsulfonate lassen sich mit Elektrolyten in wässriger Lösung nur wenig verdicken, was z.B. für die Anwendung in Shampoos nachteilig ist.

Es bestand daher ein besonderes Bedürfnis an kosmetischen Haarbehandlungsmitteln, die ein hydrolysestabiles Tensid mit guten Anwendungseigenschaften, insbesondere Schäumvermögen, Hautverträglichkeit und Verdickbarkeit in wässriger Lösung enthalten.

Es wurde gefunden, daß sich Alkylethersulfonate, insbesondere solche der nachstehend näher definierten Struktur, besonders gut für die Herstellung solcher Haarbehandlungsmittel eignen:

Gegenstand der Erfindung sind wässrige kosmetische Haarbehandlungsmittel mit anionischem Tensid, dadurch gekennzeichnet, daß als anionisches Tensid ein Alkylethersulfonat der allgemeinen Formel 1

$$R^1\text{-}O\text{-}(C_nH_{2n}O)_x\text{-}(CH_2)_y\text{-}SO_3^{(-)} M^{(+)} \qquad (1)$$

enthalten ist, in der $R^1$ eine Alkylgruppe mit 8 - 18 C-Atomen, n = 2 oder 3, x = 0 bis 20, y = 1 bis 3 und $M^{(+)}$ ein Alkaliion ist. Bevorzugt ist das Alkylethersulfonat als einziges anionisches Tensid in einer Menge von 1 bis 20 Gew.-% des Mittels enthalten, wenn das Haarbehandlungsmittel sauer ist und der pHWert in einem Bereich von ca. 1 bis 5 liegt.

Für die erfindungsgemäßen Haarbehandlungsmittel geeignete Alkylethersulfonate sind aus der Literatur bekannt und z.B. nach folgenden verschiedenen Herstellverfahren zugänglich:

a) Umsetzung von Alkylpolyglycoletherchloriden der allgemeinen Formel

$$R^1\text{-}O\text{-}(C_nH_{2n}O)_x\text{-}(CH_2)_2\text{-}Cl$$

mit Natriumsulfit, z.B. gemäß EP-A-26 932

b) Umsetzung von Alkylpolyglycolethersulfaten der allgemeinen Formel

$$R^1\text{-}O\text{-}(C_nH_{2n}O)_x\text{-}(CH_2)_2\text{-}OSO_3^{(-)}Na^{(+)}$$

mit Natriumsulfit, z.B. gemäß US-PS-3.827.497

c) Umsetzung von Alkylpolyglycolether-Alkoholaten, z.B. der Formel

$$R^1\text{-}O\text{-}(C_nH_{2n}O)_x Na$$

mit Propansulton, z.B. gemäß DE-OS 27 24 442, oder mit 2-Brom-ethansulfonsäuren oder Na-isethionat.

d) Umsetzung von Alkylpolyglycolethern der Formel

$$R^1\text{-}O\text{-}(C_nH_{2n}O)_x\text{-}H$$

mit Allylhalogenid zum Allylether der Formel

$$R^1\text{-}O\text{-}(C_nH_{2n}O)_x\text{-}CH_2\text{-}CH = CH_2$$

und Umsetzung des Allylethers mit Natriumbisulfit, z.B. gemäß EP-A-64 384.

In den vorgenannten Formeln haben R¹, n und x die gleiche Bedeutung wie in Formel 1.

Die nach den vorbeschriebenen Verfahren hergestellten Alkylethersulfonate fallen in der Regel als Natriumsalz an. Sie können aber auch (z.B. bei Verwendung von Kaliumsulfit in der Reaktion gemäß a) oder b)) auch als Kaliumsalz vorliegen. Geeignet für die erfindungsgemäßen Haarbehandlungsmittel sind aber auch Lithium-, Magnesium-, Monoethanolammonium-, Triethanolammonium-und Isopropanolammonium-Salze der Alkylethersulfonate, die man aus den Alkalisalzen auf bekannte Weise gewinnen kann.

Bevorzugt werden die Alkylethersulfonate der allgemeinen Formel I eingesetzt, in welchen y = 2 und $M^{(+)}$ ein Natrium-Ion ist. Solche Alkylethersulfonate lassen sich besonders elegant analog b) aus Alkylethersulfaten durch Umsetzung mit Natriumsulfit in wässriger Lösung unter Druck bei 150 bis 200 °C herstellen. Es wurden Verfahrensbedingungen gefunden, die eine weitgehende Umsetzung zum Alkylethersulfonat und eine anschließende Abtrennung des Tensids vom Natriumsulfat und überschüssigem Natriumsulfit ermöglichen.

Ein weiterer Gegenstand der Erfindung ist daher ein Verfahren zur Herstellung von Alkylethersulfonaten der Formel I, in der R¹ eine Alkylgruppe mit 8 - 18 C-Atomen, n = 2 oder 3, x = 0 bis 20, y = 2 und $M^{+}$ ein Natrium-Ion ist, durch Umsetzung eines Alkylethersulfats der Formel $R^1\text{-}O(C_nH_{2n}O)_x\text{-}(CH_2)_2\text{-}OSO_3^{(-)}Na^{(+)}$ mit Natriumsulfit, dadurch gekennzeichnet, daß man die Umsetzung in wässriger Lösung mit 1,5 - 4,0 Mol Natriumsulfit pro Mol Alkylethersulfat bei 150 - 250 °C unter Druck durchführt und das Reaktionsprodukt mit einem gesättigtem Alkohol mit 4 - 6 C-Atomen aus dem Reaktionsgemisch extrahiert.

Besonders geeignet für die Extraktion des Alkylethersulfonats sind n-Butanol und n-Hexanol.

Als Nebenreaktion tritt in geringerem Umfang Hydrolyse des Alkylethersulfats ein. Die dabei gebildeten unsulfierten Anteile werden zusammen mit dem Alkylethersulfonat aus der wässrigen Lösung extrahiert. Die nach dem erfindungsgemäßen Verfahren hergestellten Alkylethersulfonate enthalten daher etwa 10 - 30 Gew.-% solcher unsulfierter Anteile.

Die erfindungsgemäßen Haarbehandlungsmittel zeichnen sich durch ähnlich gute Anwendungseingenschaften aus wie solche, die mit Alkylethersulfaten hergestellt wurden, insbesondere durch gute Schäumeigenschaften, gute Verdickbarkeit, gute Haut- und Schleimhautverträglichkeit. Darüberhinaus sind die Produkte auch bei niedrigen pH-Werten lagerstabil.

Zur Herstellung der Haarbehandlungsmittel können zusätzlich zu den Alkylethersulfonaten die für die spezielle Zubereitung erforderlichen bzw. üblichen Hilfs- und Zusatzmittel eingesetzt werden, ohne daß es zu Problemen wegen mangelnder Verträglichkeit mit den Alkylethersulfonaten kommt. So können Shampoos weitere hydrolysestabile Tenside enthalten, z.B. nichtionogene Alkylpolyglycolether mit 10 - 18 C-Atomen in der Alkylgruppe und 6 - 20 Glycolethergruppen oder Alkylphenolpolyglycolether mit 8 - 12 C-Atomen in der Alkylgruppe und 8 - 20 Glycolethergruppen.

Bevorzugt enthalten erfindungsgemäße Haarbehandlungsmittel die Alkylethersulfonate der Formel I als einziges anionisches Tensid in einer Menge von 1 - 20 Gew.-% bezogen auf das gesamte Mittel. Der pH-Wert solcher Haarbehandlungsmittel kann bevorzugt im Bereich von 1 - 5 liegen.

Wenn es sich bei den erfindungsgemäßen Haarbehandlungsmitteln um Shampoos oder Duschpräparate handelt, die einen starken, gegen Fettbelastung stabilen Schaum bilden müssen, so empfiehlt sich ein zusätzlicher Gehalt an ampholytischen oder zwitterionischen Tensiden oder Aminoxid-Tensiden, bevorzugt in einer Menge von 10 - 50 Gew.-% bezogen auf das Gewicht der Aniontensids.

Als ampholytische Tenside eignen sich z.B. N-Alkyl-ß-aminopropionsäuren, N-Alkyl-ß-imminodipropionsäuren, N-Hydroxyethyl-N-alkylamidopropyl-glycin, N-Alkyl-taurine, mit jeweils 8 - 18 C-Atomen in der Alkylgruppe. Als zwitterionische Tenside eignen sich z.B.Betaintenside wie N-Alkyl-N,N-dimethyl-glycin, N-Alkylamidopropyl-N,N-dimethyl-glycin mit jeweils 8 - 18 C-Atomen in der Alkylgruppe. Als Aminoxid-Tenside eignen sich z.B. N-Kokosamidopropyl-N,N-dimethylamin-Oxid oder N-Kokosalkyl-N,N-di-(2-hydroxy)-ethylamin-oxid.

Besonders bevorzugt eignen sich die Alkylethersulfonate der Formel I als Basistenside für saure Pflegeshampoos für dauergewelltes und coloriertes Haar sowie zur Herstellung von wässrigen Haarbehandlungsmitteln mit einem Gehalt von 1 bis 10 Gewichtsprozent Wasserstoffperoxid, wie sie z.B. als Entwickler für Oxidationshaarfärbemittel oder als saure Fixierlösung nach der Dauerwellbehandlung verwendet werden. Solche Lösungen sind bevorzugt schwach sauer eingestellt z.B. durch einen Zusatz von Zitronensäure, Weinsäure, Phosphorsäure oder Organophosphonsäuren.

Außer den kennzeichnenden Alkylethersulfonaten und den vorgenannten Komponenten können die erfindungsgemäßen Haarbehandlungsmittel alle für solche Zubereitungen üblichen Bestandteile enthalten. Hierzu gehören insbesondere nichtionische, ampholytische, zwitterionische und Aminoxid-Tenside. Weiterhin auch Duftstoffe, kosmetische Öl- und Fettkomponenten, Fettalkohole, Wachse, Puffersalze, Konservierungsstoffe und Farbstoffe.

Die folgenden Beispiele sollen den Gegenstand der Erfindung näher erläutern ohne ihn hierauf zu beschränken.

**Beispiele**

1. Herstellungsbeispiele

1.1 Herstellung Von Alkyl ($C_{12}$-$C_{14}$) + 2 EO-sulfonat-Na-Salz 10 kg einer 70 %igen, wässrigen Alkylethersulfat-Natriumsalz-Paste (Basis Lauryl-myristyl (70:30)-alkohol + 2 Mol Ethylenoxid) wurden zusammen mit 7,7 kg Natriumsulfit und 26,6 kg Wasser in einem Autoklaven 3 Stunden auf 200 °C erhitzt. Nach dem Abkühlen auf 25 °C wurde das Reaktionsgemisch dreimal mit n-Butanol extrahiert und das n-Butanol aus dem Extrakt abdestilliert. Es wurden 6,0 kg eines wachsartigen Festproduktes mit einem Gehalt von 1,83 mval/g Aniontensid (bestimmt nach DGF-Einheitsmethoden H-III-10) * und 18,4 Gewichtsprozent unsulfierten Anteilen erhalten.

1.2 Herstellung von Alkyl ($c_{12}$-$c_{14}$) + 3,6 EO-sulfonat-Na-Salz Analog Beispiel 1 wurden 20,3 kg einer 30 %igen, wässrigen Alkylethersulfat-Natriumsalz-Lösung (Basis Lauryl-myristyl (50:50)-alkohol + 3,6 Mol Ethylenoxid) mit 4,9 kg Natriumsulfit und 14,8 kg Wasser 3 Stunden auf 200 °C erhitzt.

Nach Aufarbeitung wurden 5,6 kg eines wachsartigen Festproduktes mit einem Gehalt von 1,63 mval/g Aniontensid (bestimmt nach DGF-Einheitsmethode H-III-10) * und 20 Gewichtsprozent unsulfierten Anteilen erhalten.

1.3 Herstellung von Alkyl-($C_{12}$-$C_{15}$) + 12 EO-sulfonat, Na-Salz

Analog Beispiel 1 wurden 990 g einer 30 %igen wässrigen Lösung eines Alkylethersulfat-Natriumsalzes (Basis Oxoalkohol $C_{12}$-$C_{15}$ + 12 Mol Ethylenoxid) mit 160 g Natriumsulfit und 3500 g Wasser 3 Stunden auf 200 °C erhitzt.

Nach Aufarbeitung wurden 250,7 g eines wachsartigen Festproduktes mit einem Gehalt von 0,844 mval/g Aniontensid (nach DGF-Einheitsmethode H-III-10) erhalten.

2. Anwendungsbeispiele

2.1 Saures Shampoo

| | | |
|---|---|---|
| Alkylethersulfonat nach Beispiel 1.1 | 10 | Gew.% |
| $C_{12}$-$_{18}$-Acylaminopropyl-dimethyl-ammoniumglycinat | 3 | Gew.% |
| Parfümöl | 0,2 | Gew.% |
| Bronidox $^R$ L (Konservierungsmittel) | 0,2 | Gew.% |
| Zitronensäure | bis pH = 3 | |
| Wasser | ad 100 | Gew.% |

2.2 Saure Dauerwellenfixierlösung

* Die Bestimmung des Aniontensidgehalts nach DGF-Einheitsmethode H-III-10 erfolgte nach der sogenannten Zweiphasentitrationsmethode. Vorher wurden durch saure Hydrolyse (2nHCl) die hydrolysierbaren Anteile von Alkylethersulfat entfernt.

4

| | | |
|---|---|---|
| Alkylethersulfonat nach Beispiel 1.1 | 4 | Gew.% |
| Zitronensäure | 0,5 | Gew.% |
| Wasserstoffperoxid, 30 %ige Lösung | 5 | Gew.% |
| Natriumpyrophosphat | 0,5 | Gew.% |
| Parfümöl | 0,3 | Gew.% |
| Wasser | ad 100 | Gew.% |

2.3 Entwicklerdispersion für Oxidationshaarfärbemittel

| | | |
|---|---|---|
| Cetylalkohol | 2 | Gew.% |
| Alkylethersulfonat nach Beispiel 1.3 | 3 | Gew.% |
| Fettalkohol ($C_{12}$–$C_{14}$) + 3 Mol Ethylenoxid | 2 | Gew.% |
| Wasserstoffperoxid (30 %ige Lösung) | 20 | Gew.% |
| Hydroxyethandiphosphonsäure | 0,5 | Gew.% |
| Wasser | ad 100 | Gew.% |

**Patentansprüche**

1. Wässrige Haarbehandlungsmittel mit üblichen Bestandteilen, Hilfs-und/oder Zusatzstoffen sowie anionischem Tensid, dadurch gekennzeichnet, daß als anionisches Tensid ein Alkylethersulfonat der allgemeinen Formel

$R^1$- O - $(C_nH_{2n}O)_x$ - $(CH_2)_y$ - $SO_3(-)M(+)$

enthalten ist, in der $R^1$ eine Alkylgruppe mit 8 - 18 C-Atomen, n = 2 oder 3, x = 0 bis 20, y = 1 bis 3 und M( + ) ein Alkali-Ion ist.

2. Haarbehandlungsmittel nach Anspruch 1, dadurch gekennzeichnet, daß y = 2 und $M^{(+)}$ ein Natrium-Ion ist.

3. Haarbehandlungsmittel nach Anspruch 1 oder 2, dadurch gekennzeichnet, daß das Alkylethersulfonat als einziges anionisches Tensid in einer Menge von 1 - 20 Gewichtsprozent enthalten ist und der pH-Wert des Mittels bei 1 - 5 liegt.

4. Haarbehandlungsmittel nach Anspruch 1 - 3, dadurch gekennzeichnet, daß es zusätzlich ein amphoteres oder zwitterionisches Tensid in einer Menge von 10 - 50 Gewichtsteilen bezogen auf das Gewicht des Aniontensids enthält.

5. Haarbehandlungsmittel nach Anspruch 1 - 3, dadurch gekennzeichnet, daß es zusätzlich 1 - 10 Gewichtsprozent Wasserstoffperoxid enthält.

6. Verfahren zur Herstellung von Alkylethersulfonaten der Formel

(I)     $R^1$-O $(C_nH_{2n}O)_x$ - $(CH_2)_y$ - $SO_3^{(-)}M^{(x)}$

in der $R^1$ eine Alkylgruppe mit 8 - 18 C-Atomen, n = 2 oder 3, x 0 bis 20, y = 2 und $M^{(+)}$ ein Natrium-Ion ist, durch Umsetzung eines Alkylethersulfats der Formel

$R^1$-O $(C_nH_{2n}O)_x$ - $(CH_2)_2$ - O - $SO_3^{(-)}Na^{(+)}$

EP 0 251 250 B1

mit Natriumsulfit, dadurch gekennzeichnet, daß man die Umsetzung in wassriger Lösung mit 1,4 - 4,0 Mol Natriumsulfit pro Mol Alkylethersulfat bei 150 - 250 °C unter Druck durchführt und das Reaktionsprodukt mit einem gesättigten Alkohol mit 4 - 6 C-Atomen aus dem Reaktionsgemisch extrahiert.

**Claims**

1. Aqueous hair treatment preparations containing typical constituents, auxiliaries and additives and also an anionic surfactant, characterized in that the anionic surfactant present is an alkyl ether sulfonate corresponding to the following general formula

   $R^1$-O-$(C_nH_{2n}O)_x$-$(CH_2)y$-$SO_3^{(-)}M^{(+)}$  (I)

   in which $R^1$ is a $C_{8-18}$ alkyl group, n = 2 or 3, x = 0 to 20, y = 1 to 3 and $M^{(+)}$ is an alkali ion.

2. Hair treatment preparations as claimed in claim 1, characterized in that y = 2 and $M^{(+)}$ is a sodium ion.

3. Hair treatment preparations as claimed in claim 1 or 2, characterized in that the alkyl ether sulfonate is present as sole anionic surfactant in a quantity of from 1 to 20% by weight and the pH value of the preparation is in the range from 1 to 5.

4. Hair treatment preparations as claimed in claims 1 to 3, characterized in that they additionally contain an amphoteric or zwitterionic surfactant in a quantity of 10 to 50 parts by weight, based on the weight of the anionic surfactant.

5. Hair treatment preparations as claimed in claims 1 to 3, characterized in that they additionally contain 1 to 10% by weight hydrogen peroxide.

6. A process for the production of alkyl ether sulfonates corresponding to the following formula

   $R^1$-O-$(C_nH_{2n}O)_x$-$(CH_2)_y$-$SO_3^{(-)}M^{(+)}$  (I)

   in which $R^1$ is a $C_{8-18}$ alkyl group, n = 2 or 3, x = 0 to 20, y = 2 and $M^{(+)}$ is an sodium ion,
   by reaction of an alkyl ether sulfate corresponding to the following formula

   $R^1$-O $(C_nH_{2n}O)_x$-$(CH_2)$ $_2$-O-$SO_3^{(-)}Na^{(+)}$

   with sodium sulfite, characterized in that the reaction is carried out under pressure at 150-250 °C in aqueous solution with 1.4 to 4.0 mol sodium sulfite per mol alkyl ether sulfate and the reaction product is extracted from the reaction mixture with a saturated $c_{4-6}$ alcohol.

**Revendications**

1. Composition aqueuse de traitement des cheveux avec les constitutants, les substances auxiliaires et les additifs usuels, ainsi qu'un agent tensioactif anionique, caractérisée en ce qu'en tant qu'agent tensioactif anionique elle renferme un alcoyléthersulfonate de formule générale

   $R^1$-O-$(C_nH_{2n}O)_x$-$(CH_2)_y$-$SO_3^{(-)}M^{(+)}$

   dans laquelle $R_1$ est un radical alcoyle ayant de 8 à 18 atomes de carbone
   n = 2 ou 3
   x = 0 à 20
   y = 1 à 3
   et $M^{(+)}$ est un ion alcalin.

2. Composition de traitement des cheveux selon la revendication 1, caractérisée en ce que y = 2 et $M^{(+)}$ est un ion sodium.

3. Composition de traitement des cheveux selon les revendications 1 ou 2, caractérisée en ce que l'acoyléthersulfonate est contenu en tant qu'agent tensioactif anionique unique en quantité allant de 1 à 20 % en poids et que la valeur du pH de la composition se situe de 1 à 5.

4. Composition de traitement des cheveux selon les revendications 1 à 3, caractérisée en ce qu'elle renferme en addition un agent tensioactif amphotère ou zwitterionique en quantité allant de 10 à 50 parties en poids rapporté au poids d'agent tensioactif anionique.

5. Composition de traitement des cheveux selon les revendications 1 à 3, caractérisée en ce qu'elle renferme en outre de 1 à 10 % en poids de peroxyde d'hydrogène.

6. Procédé d'obtention des alcoyléthersulfonates de formule

   (I)　　$R^1\text{-O-}(C_nH_{2n}O)_x\text{-}(CH_2)_y\text{-}SO_3^{(-)}M^{(+)}$

   dans laquelle $R^1$ est un radical alcoyle ayant de 8 à 18 atomes de carbone
   n = 2 ou 3
   x = 0 à 20
   y = 2
   et $M^{(+)}$ est un ion sodium
   par réaction d'un alcoyléthersulfate de formule

   $R^1\text{-O-}(C_nH_{2n}O)_x\text{-}(CH_2)_2\text{-O-}SO_3^{(-)}Na^{(+)}$

   avec du sulfite de sodium
   caractérisé en ce que l'on effectue la réaction en solution aqueuse avec 1,4 à 4,0 mol de sulfite de sodium par mol d'alcoyléthersulfate à 150 - 250 °C sous pression et que l'on extrait le produit de la réaction du mélange réactionnel avec un alcool saturé ayant de 4 à 6 atomes de carbone .